# EUROPÄISCHE PATENTANMELDUNG

(11) **EP 1 679 081 A2**
(43) Veröffentlichungstag der Anmeldung: **12.07.2006**
(21) Anmeldenummer: 06006105.8
(22) Anmeldetag: 02.05.2003
(51) Int. Cl.: A61K 38/10

(54) **Therapeutische Verwendung von Cephaibolen**

(30) Priorität: 23.05.2002 DE 10222792
(62) Teilanmeldung aus: 03722587.7
(71) Anmelder: Sanofi-Aventis Deutschland GmbH, 65929 Frankfurt am Main (DE)
(72) Erfinder: Schiell, Matthias, Di., 65611 Brechen (DE); Vértesy, Laszlo, Dr., 65817 Eppstein-Vockenhausen (DE); Wink, Joachim, Dr., 63322 Rödermark (DE); Schlegel, Brigitte, DR., 07743 Jena (DE); Haertl, Albert, Dr., 07749 Jean (DE); Graefe, Udo, Prof. Dr., 07745 Jean (DE)

(57) **Zusammenfassung**

Die Erfindung betrifft die Verwendung von Verbindungen der allgemeinen Formel I AcPhe-Aib-Aib-Aib-x-w-Leu-y-Aib-Hyp-Gln-z-Hyp-Aib-Pro-R (I) in der R Phe-ol oder Phe-al bedeutet und w, x, y, und z folgende Bedeutung haben:
a) w gleich Gly oder Ala; x gleich Aib und y und z gleich Iva;
b) w gleich Gly; x, y und z gleich Iva;
c) w gleich Gly; x und z gleich Aib und y gleich Iva;
d) w gleich Gly; x, y und z gleich Aib; oder
e) w gleich Gly; x und y gleich Aib und z gleich lva;
oder der allgemeinen Formel II AcPhe-Iva-Gln-Aib-Ile-Thr-Aib-Leu-Aib-x-Gln-Aib-Hyp-Aib-Pro-Phe-Ser (II) in der x Hyp oder Pro bedeutet, sowie deren physiologisch verträglichen Salze zur Herstellung eines Arzneimittels mit neurologischer und/oder immunsuppremierender Wirkung.

## Beschreibung

Die Erfindung betrifft die Verwendung von Cephaibolen zur Herstellung von Arzneimitteln mit neurologischer sowie immunsuppremierender Wirkung.

Peptide mit bis zu 20, zum Teil strukturell ungewöhnlichen Aminosäuren werden von Bakterien und Pilzen über deren Sekundärstoffwechsel mittels nichtribosomaler Peptid-Synthetases produziert. Viele der bisher bekannten Sekundärmetabolite mit Peptidstruktur besitzen interessante biologische Wirkungen als Antibiotika, Enzyminhibitoren, Kardiotonika, Immunmodulatoren, Insektizide, Nematozide u.a.m. (siehe z.B. Gräfe, U. Biochemie der Antibiotika, Spektrum Heidelberg, 1992).

Innerhalb der Strukturklasse der Peptidwirkstoffe sind die sogenannten Peptaibole dadurch ausgezeichnet, dass sie ungewöhnlich viele Aminosäuren (bis zu 20), darunter einen hohen Anteil an alpha-Aminobuttersäure enthalten (Brückner, H. , König, W.A., Greiner, M., Jung, G. Angew. Chem. Int. Ed. Engl. 18 (1979), 476 -477). Ferner sind Peptaibole sehr häufig am N-Terminus acetyliert und weisen am C-Terminus einen Rest mit einer Alkoholgruppe (z.B. Phenylalaninol) oder einer Aldehydgruppe auf.

Als Wirkungsweise der Peptaibole wird allgemein das Ausbilden von Poren in biologischen Membranen angenommen (M. K. Das et al. Biochemistry, 25, 7110-7117, 1986). Dadurch können unkontrolliert Ionen in die Zelle eindringen und die lebenswichtigen biochemischen Prozesse stören, was die antibiotische Wirkung vieler Peptaibole erklären könnte. Neben der antibiotischen Wirkung wurden für verschiedene Peptaibole weitere unterschiedliche biologische Wirkungen beobachtet. Ampullosporine induzieren die Pigment-Bildung bei *Phoma destructiva* und erzeugen neuroleptische Wirkungen in der Maus (WO99/12598; DE199148644; M. Ritzau et al. J. Antibiotics 50, 722-728, 1997; Kronen et al., J. Antibiotics, 54,175-178, 2001). Die Bergofungin-Komponenten (A. Berg et al. J. Antibiotics 52, 666-669, 1999) hemmen die Prolyl-Endopeptidase in submikromolaren Konzentrationen, haben aber keine Wirkungen auf *Phoma destructiva.* Das Clonostachin (T. Chikanishi et al. J.

Antibiotics, 50, 105-110, 1997) hemmt die Platelet Aggregation. Vor kurzem sind die antiviralen Peptaivirine A und B (B.-S. Yun et al. Tetrahedron Letters, 41, 1429-1431, 2000) beschrieben worden. Aufgrund der strukturellen Unterschiede der Peptaibole ist eine einheitliche Erklärung und Vorhersage der biologischen Aktivitäten der verschiedenen Peptaibole nicht möglich.

Eine weitere Gruppe der Peptaibol-Antibiotika stellen die Cephaibole dar, welche linear aus 16 oder 17, zum Teil nicht kodierbaren Aminosäuren oder Aminosäurederivaten aufgebaut sind. Struktur, Herstellung und biologische Aktivitäten der Cephaibole sind beispielsweise in WO 00/68256 oder M. Schiell et al., J. Antibiotics, 54 (2001) 220-233 beschrieben. Cephaibole besitzen Hemmwirkungen gegen human- und/oder tierpathogene Endo- und/oder Ektoparasiten, wie Trematoden, Nematoden, Arachnida und einige Insecta. Darüber hinaus weisen die Cephaibole antibakterielle Aktivitäten auf.

Überraschenderweise wurde nun gefunden, dass Cephaibole in verschiedenen Testmodellen weiterer Effekte zeigen, aufgrund dessen sie für eine therapeutische Anwendung als Immunsuppressiva oder Mittel mit neurologischer, insbesondere neuroleptischer Wirkung geeignet sind.
Die Erfindung betrifft somit die Verwendung von Verbindungen der allgemeinen Formel I

**AcPhe-Aib-Aib-Aib-x-w-Leu-y-Aib-Hyp-Gln-z-Hyp-Aib-Pro-R** (I)

in der R Phe-ol oder Phe-al bedeutet und w, x, y, und z folgende Bedeutung haben:
a) w gleich Gly oder Ala; x gleich Aib und y und z gleich Iva;
b) w gleich Gly; x, y und z gleich Iva;
c) w gleich Gly; x und z gleich Aib und y gleich Iva;
d) w gleich Gly; x, y und z gleich Aib; oder
e) w gleich Gly; x und y gleich Aib und z gleich Iva;
oder von Verbindungen der allgemeinen Formel II

AcPhe-Iva-Gln-Aib-Ile-Thr-Aib-Leu-Aib-x-Gln-Aib-Hyp-Aib-Pro-Phe-Ser (II)

in der x Hyp oder Pro bedeutet; sowie deren physiologisch verträglichen Salze zur Herstellung eines Arzneimittels mit neurologischer und/oder immunsuppremierender Wirkung.

Verbindungen der allgemeinen Formel I oder II werden auch als Peptidwirkstoffe oder Cephaibole bezeichnet.

Die Erfindung betrifft ferner die Verwendung der Cephaibole A, A1, B, C, D, E, P und Q.

Cephaibol A bezeichnet eine Verbindung der allgemeinen Formel I, in der R Phe-ol, w Gly x Aib und y und z lva bedeuten.

AcPhe-Aib-Aib-Aib-Aib-Gly-Leu-Iva-Aib-Hyp-Gln-Iva-Hyp-Aib-Pro-Phe-ol (SEQ ID No. 1)

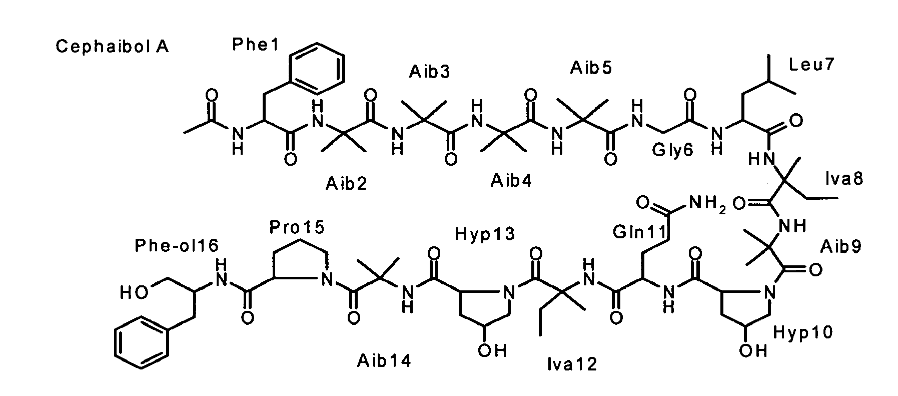

Cephaibol A1 bezeichnet eine Verbindung der allgemeinen Formel I, in der R Phe-ol, w Ala, x Aib und y und z Iva bedeutet.

AcPhe-Aib-Aib-Aib-Aib-Ala-Leu-Iva-Aib-Hyp-Gln-Iva-Hyp-Aib-Pro-Phe-ol (SEQ ID No. 8)

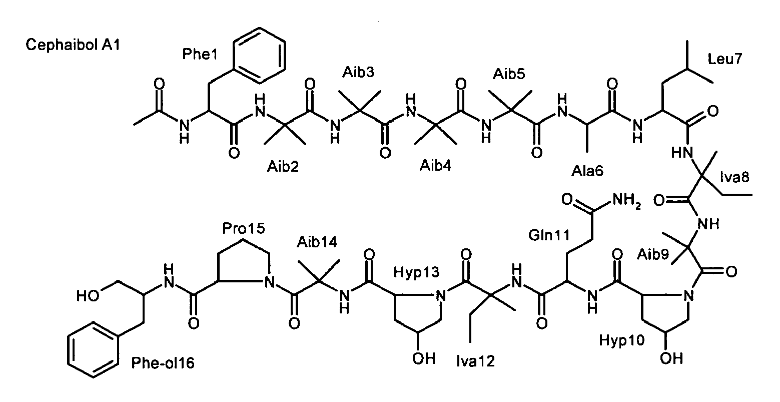

Cephaibol B bezeichnet eine Verbindung der allgemeinen Formel I, in der R Phe-ol, w Gly und x,y und z Iva bedeutet.

AcPhe-Aib-Aib-Aib-Iva-Gly-Leu-Iva-Aib-Hyp-Gln-Iva-Hyp-Aib-Pro-Phe-ol (SEQ ID No. 2)

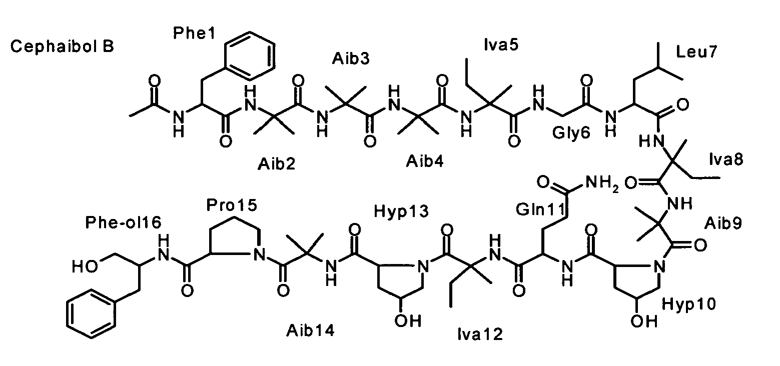

Cephaibol C bezeichnet eine Verbindung der allgemeinen Formel I, in der R Phe-ol, w Gly, x und z Aib und y Iva bedeutet.

AcPhe-Aib-Aib-Aib-Aib-Gly-Leu-Iva-Aib-Hyp-Gln-Aib-Hyp-Aib-Pro-Phe-ol (SEQ ID No. 3)

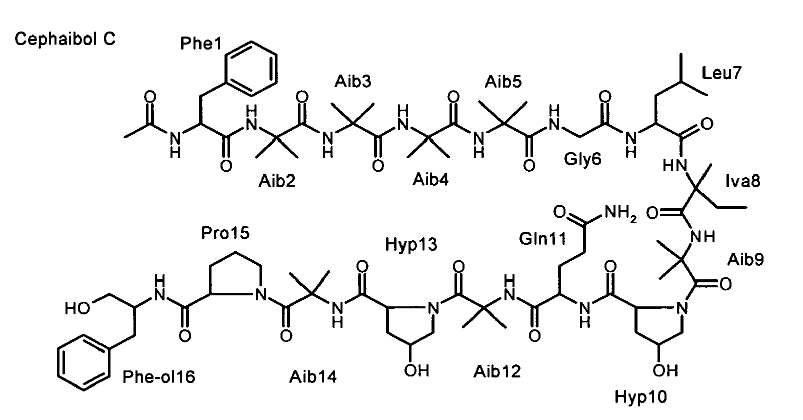

Cephaibol D bezeichnet eine Verbindung der allgemeinen Formel I, in der R Phe-ol, w Gly und x, y und z Aib bedeutet.

AcPhe-Aib-Aib-Aib-Aib-Gly-Leu-Aib-Aib-Hyp-Gln-Aib-Hyp-Aib-Pro-Phe-ol (SEQ ID No. 4)

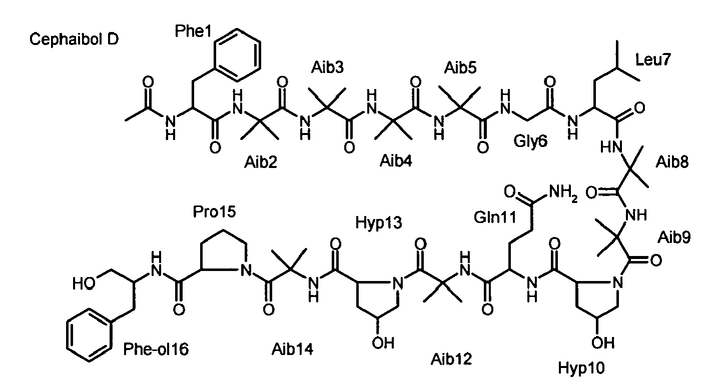

Cephaibol E bezeichnet eine Verbindung der allgemeinen Formel I, in der R Phe-ol, w Gly, x und y Aib und z Iva bedeutet.

AcPhe-Aib-Aib-Aib-Aib-Gly-Leu-Aib-Aib-Hyp-Gln-Iva-Hyp-Aib-Pro-Phe-ol (SEQ ID No. 5)

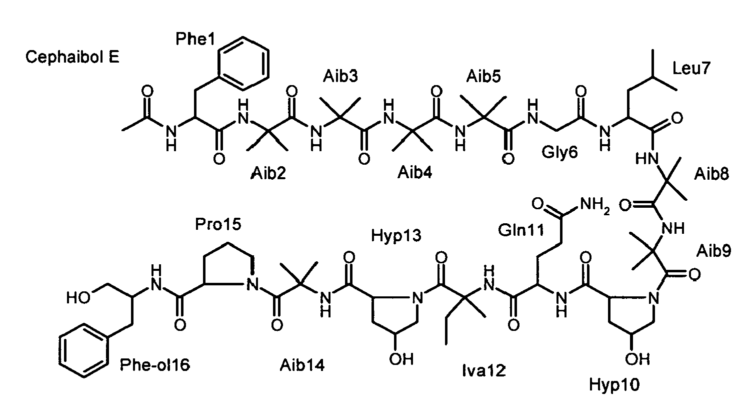

Cephaibol P bezeichnet eine Verbindung der allgemeinen Formel II, in der x Hyp bedeutet.

AcPhe-Iva-Gln-Aib-Ile-Thr-Aib-Leu-Aib-Hyp-Gln-Aib-Hyp-Aib-Pro-Phe-Ser (SEQ ID No. 6)

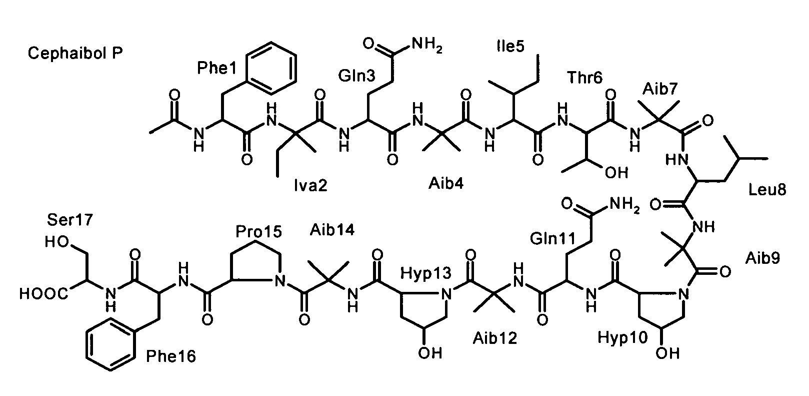

Cephaibol Q bezeichnet eine Verbindung der allgemeinen Formel II, in der X Pro bedeutet.

AcPhe-Iva-Gln-Aib-Ile-Thr-Aib-Leu-Aib-Pro-Gln-Aib-Hyp-Aib-Pro-Phe-Ser (SEQ ID No. 7)

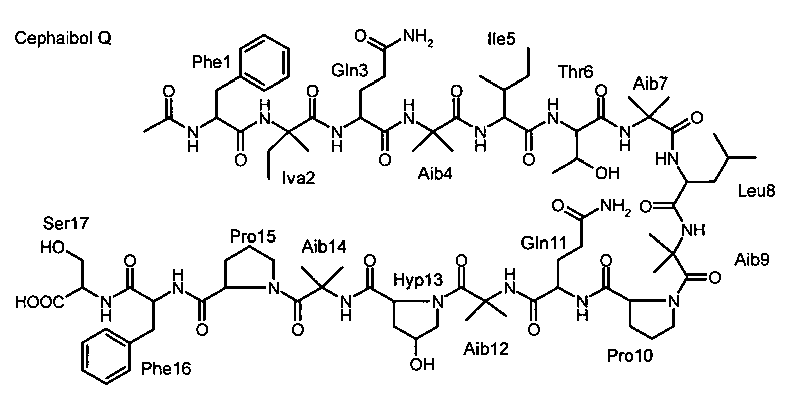

AcPhe steht für N-Acetylphenylalanin, Aib für α-Aminoisobuttersäure, Ala für Alanin, Iva für Isovalin, Hyp für Hydroxyprolin, Phe-ol für Phenylalaninol, Phe-al für Phenylalaninal, Phe für Phenylalanin, Gly für Glycin, Leu für Leucin, Gln für Glutamin, Pro für Prolin, Ile für Isoleucin, Thr für Threonin und Ser für Serin.

Die obengenannten Verbindungen sind bekannt und lassen sich beispielsweise wie in WO 00/68256 oder Schiell et al, J. Antibiotics, 54 (2001), 220-233, beschrieben, herstellen. Die obenbeschriebenem Cephaibole können beispielsweise durch den Mikroorganismus Acremonium tubakii FH 1685 DSM 12774 produziert werden, wobei der Mikroorganismus unter geeigneteten Bedingungen fermentiert wird, bis sich die Cephaioble in dem Fermentationsmedium anhäufen und anschließend isoliert und gereinigt werden können (WO 00/68256; Schiell et al, J. Antibiotics, 54 (2001), 220-233). Ein Isolat wurde bei der Deutschen Sammlung von Mikroorganismen und Zellkulturen GmbH, Mascheroder Weg 1 B, D 38124 Braunschweig, Deutschland, nach den Regeln des Budapester Vertrages am 31. März 1999 unter der folgenden Nummer hinterlegt: Acremonium tubakii FH 1685 DSM 12774.

Die Erfindung bezieht sich auf die Verwendung der Cephaibole der Formel I und II in Form ihrer Racemate, racemischen Mischungen, und reinen Enatiomeren sowie auf ihre Diasteromere und Mischungen davon. Sofern die obengenannten Verbindungen diastereoisomere oder enatiomere Formen zulassen und bei der gewählten Synthese als deren Gemische anfallen, gelingt die Trennung in die reinen Stereoisomeren entweder durch Chromatograhie an einem gegebenenfalls chiralen Trägermaterial oder, sofern die racemischen obengenannten Verbindungen zur Salzbildung befähigt sind, durch fraktionierte Kristallisation der mit einer optisch aktiven Base oder Säure als Hilfsstoff gebildeten diastereomeren Salze.

Die vorliegende Erfindung umfasst auch die Verwendung offensichtlich chemische Äquivalente der Verbindungen der Formel I oder II. Solche sind z.B. Ester, Ether, Additionssalze, Komplexe, oder auch Partialhydrolyseprodukte.

Unter physiologisch verträglichen Salzen von Verbindungen der Formel I oder II versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die Erfindung betrifft auch pharmazeutische Zubereitungen, die eine oder mehrere der Cephaibole enthalten. Bevorzugt ist die Verwendung in Mischung mit geeigneten Hilfsstoffen oder Trägermaterial. Als Trägermaterial können alle pharmakologisch verträglichen Trägermaterialien und/oder Hilfsstoffe verwendet werden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Die Arzneimittel werden im allgemeinen oral, lokal oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Cephaibole enthält. Bei festen Dosierungseinheiten wie Tabletten, Kapseln und Suppositorien kann diese Dosis bis zu etwa 2000 mg, bevorzugt jedoch etwa 1 bis 1000 mg, und bei Injektionslösungen in Ampullenform bis zu etwa 1000 mg, vorzugsweise aber etwa 10 bis 300 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die Erfindung betrifft auch ein Verfahren zur Herstellung eines Arzneimittels, das dadurch gekennzeichnet ist, daß man die Cephaibole mit üblichen Träger- sowie gegebenenfalls Zusatz- und/oder Hilfstoffe in die bzw. eine geeignete Darreichungsform bringt.

Störungen des Nervensystems, insbesondere des zentralen Nervensystems sind die Ursache von mannigfaltigen Erkrankungen. Es sind unter anderem Erkrankungen, die mit psychopathologischen Symptomen, wie Halluzinationen, Wahn, psychomotorischen Erregengen oder mit anderen Erscheinungen einhergehen. Als Beispiele seien hier die Schizophrenie, die Epilepsie oder die Parkinson'sche Krankheit genannt, es sind aber zahlreichen weitere Erkrankungen bekannt, die entweder die Steuerung von Körperfunktionen betreffen oder häufig auch psychotischer Natur sind. Es sind gewöhnlich progressive, das heißt in ihrem Verlauf fortschreitende Krankheiten. Ihre medikamentöse Behandlung geschieht mit Psychopharmaka, die z. B. Neuroleptika, Antidepressiva oder Tranquillantien umfassen. Ihre Zweckbestimmung besteht darin, die psychopathologische Symptome zu beseitigen oder abzuschwächen. Tranquillantien sind Verbindungen mit vorwiegend dämpfender Wirkung auf die Psyche, die die Angst vermindern. Antidepressiva sind Stoffe, die eine pathologisch gesenkte Grundstimmung heben und depressive Wahngedanken beseitigen können. Neuroleptika sind geeignet, um psychomotorische Erregung, affektive Erregbarkeit und Vigilanz zu dämpfen und Antrieb, Spontanbewegungen und Ausdrucksmotorik zu vermindern, wobei die intellektuellen Fähigkeiten erhalten bleiben sollen.

Die obengenannten Cephaibole zeigen in in vivo Versuchen an Mäusen neuroleptische Effekte, wie z.B. die Reduktion der spontanen Motilität oder eine Hypothermie. Aufgrund dieser beobachteten pharmakologischen Effekte wird angenommen, dass die Verbindungen der Formel I und II und insbesondere die Cephaibole A, A1, B, C, D, E, P und Q als Agenzien verwendet werden können, die auf das zentrale oder periphere Nervensystem wirken und für die Behandlung von neurologischen Erkrankungen geeignet sind, beispielsweise zur Behandlung von Krankheiten oder Krankheitszuständen, bei denen Psychopharmaka wie Neuroleptika, Antidepressiva oder Tranquillantien , insbesondere Neuroleptika Anwendung finden.

Dazu gehört beispielsweise die Anwendung bei Halluzinationen, Wahnvorstellungen, psychomotorischer Erregtheit, ängstlicher Agitiertheit, Schizophrenie, akuten manischen Phasen, akut psychotischen Syndromen wie beispielsweise paranoide oder paranoid-halluzinatorische Zustände, Angst- und Spannungszuständen aber auch die Verwendung bei der Narkoseprämedikation, Neuroleptanalgesie bzw. Neuroleptanästhesie oder auch der Einsatz bei Erbrechen.

Des weiteren induzieren die oben beschriebenen Cephaibole die Pigmentbildung in Phoma Destructiva in Ähnlicherweise wie das bekannte Cyclosporin A, das ein wirksames Immunsuppressivum ist. Die Verbindungen der Formel I und II und insbesondere die Cephaibole A, A1, B, C, D, E, P und Q sind somit auch zur Anwendung bei Krankheiten und Zuständen geeignet, bei denen Immunsuppressiva Einsatz finden, insbesondere in der Transplantationsmedizin oder bei Autoimmunkrankheiten. Dazu gehört beispielsweise die Anwendung bei chronischer Glomerulonephritis mit nephrotischen Syndrom, chronisch entzündlichen Darmkrankheiten, Myasthenia gravis, Autoimmunhepatitis, thrombopenische Purpura oder entzündliche rheumatische Erkrankungen wie beispielsweise Dermatomyositis, Lupus erythematodes, rheumatoide Arthritis oder Sklerodermie.

Unter Behandlung von Krankheiten oder Krankheitszuständen ist auch die prophylaktische Behandlung zur deren Verhinderung oder verlangsamten Auftreten zu verstehen.

### Die Wirksamkeit der Cephaibole wurde wie folgt getestet:

### Neuroleptische Wirkung der Cephaibole im Maus-Modell

### a) Einfluss auf spontane Motilität

Der Einfluss der Cephaibole A, B, C, D und E auf die Motorik wurde über verschiedene Zeiträume (von 5 min bis zu 24 Stunden) an Labormäusen beobachtet. Nach intraperitonealer Verabreichung von jeweils 10mg/kg Cephaibol A, B, C, D und E war im Gegensatz zu den Kontrollmäusen unten den gewählten Versuchsbedingungen nach unterschiedlichen Beobachtungszeiträumen eine Beeinträchtigung der Motorik (Hangeln) zu beobachten. Besonders ausgeprägt waren die beobachteten Effekte nach Gabe der Cephaibole B und C.
Die beobachtet Hemmung der Motorik verweist auf neuroleptische Wirkqualitäten der getesteten Cephaibole. Schreckreaktionen (auf Geräusche) blieben erhalten, was eine narkotische Wirkung der Cephaibole in der Versuchsanordnung ausschließt.

### b) Hypothermie

Die intraperitoneale Verabreichung von jeweils 10mg/kg Cephaibol A, B, C und E an Labormäusen bewirkte im Gegensatz zu den Kontrollmäusen unter den gewählten Versuchsbedingungen eine über einen längeren Zeitraum beobachtbare deutliche Absenkung der Körpertemperatur (besonders ausgeprägt bei den Cephaibolen B und C), was ebenfalls auf neuroleptische Wirkqualitäten der getesteten Cephaibole verweist.

### Immunsuppremierende Wirkung im Phoma destructiva -Modell

Die Induktion der Pigmentbildung des Pilzes Phoma destructiva ist ein Testmodell um unter anderen eine potentielle immunsuppremierende Wirkung von Testsubstanzen zu erkennen. Die Durchführung des *Phoma destructiva-*Testes ist von K. Dornberger et al. im J. Antibiotics, 48, 977-989, 1995 beschrieben worden.

Der Organismus *Phoma destructiva* ist ein bekannter pflanzenpathogener Pilz, der auf Agarplatten, angeimpft mit vegetativem Mycel, in farbschwachen Kolonien wächst. Unter Einfluß von speziellen Induktoren werden Zelldifferenzierungs-vorgänge ausgelöst, die mit einer Pigment-Bildung einhergehen und daher die Zelldifferenzierung erkennen lassen. Das Pigment, eine melaninähnliche Substanz, kann an der Färbung erkannt und photometrisch oder durch Messen der Zonengröße quantitativ erfaßt werden. Das bekannte Immunsupressivum Cyclosporin A bewirkt eine morphologische Veränderung und Pigmentbildung im Testorganismus.
Es wurde nun gefunden das die erfindungsgemäßen Cephaibole in Phoma Destructiva eine ähnliche Pigmentbildung wie das Cyclosporin A hervorrufen.

### Versuchsanordnung:

Der Pilz *Phoma destructiva* wurde auf Schrägagar-röhrchen mit dem Kulturmedium A oder B bei 20 bis 25° C kultiviert, anschließend bei 4 bis 6° C aufbewahrt und im 2-Jahresrythmus überimpft.
- Kulturmedium A:: Malzextrakt 40 g/L,
Hefeextrakt 4 g/L,
Agar 15 g/L.
- Kulturmedium B:: Kartoffel-Glucose-Agar (E. Merck) 39 g/L.

### Impfkultur:

Der Pilz *Phoma destructiva* wurde 11 Tage bei 20 bis 25° C auf dem Medium B in Petrischalen mit 100 mm Durchmesser kultiviert, danach mit 10 mL einer sterilen 0.9%-igen Lösung von NaCl abgeschwemmt. Die Abschwemmung kann bei 6° C - Lagerung 5 Tage verwendet werden.

### Lochplatten-Agardiffusionstest:

Die Testmethode des Agardiffusionstestes wurde in Anlehnungan den standardisierten Agardiffusionsassay des Europäischen Arzneibuches [Deutscher Apothekerverlag Stuttgart, Seite 113-118, 1997] variiert: 40 mL Testagarmedium wurden verflüssigt, auf 50° C temperiert, 8 bis 10 mL der Impfkultur steril hinzugefügt und anschließend in Testplatten mit 150 mm Durchmesser gegossen. Nach Erkalten wurden 12 Stanzlöcher mit Durchmessern von 9 mm mit einem Stanzgerät auf der Platte angeordnet [J, V. Benett et al. Appl. Micr. 14, 170-177, 1966]. Die Testlöcher wurden mit 50 µL der zu prüfenden Lösung beschickt wobei ein Testloch Cyclosporin als Kontrollsubstanz (1 µg Cyclosporin A in 50 µL Methanol) enthielt. Die so präparierten Platten wurden 4 bis 5 Tage bei 20 bis 25° C kultiviert.
Die Cephaibole verursachen unter den oben genannten Testbedingungen eine Pigmentbildung, die der gelben Pigmentierung des Cyclosporins A ähnelt. Besonders ausgeprägt war dieser Effekt bei den Cephaibolen A, B, und C.

## Patentansprüche

1. Verwendung von Verbindung der Formel I,
**AcPhe-Aib-Aib-Aib-x-w-Leu-y-Aib-Hyp-Gln-z-Hyp-Aib-Pro-R** (I)
in der R Phe-ol oder Phe-al bedeutet und w, x, y, und z folgende Bedeutung haben:
a) w gleich Gly oder Ala; x gleich Aib und y und z gleich Iva;
b) w gleich Gly; x, y und z gleich Iva;
c) w gleich Gly; x und z gleich Aib und y gleich Iva;
d) w gleich Gly; x, y und z gleich Aib; oder
e) w gleich Gly; x und y gleich Aib und z gleich Iva;
oder von Verbindungen der Formel II
**AcPhe-lva-Gln-Aib-Ile-Thr-Aib-Leu-Aib-x-Gln-Aib-Hyp-Aib-Pro-Phe-Ser** (II)
in der x Hyp oder Pro bedeutet,
sowie deren physiologisch verträglichen Salze zur Herstellung eines Arzneimittels mit immunsuppremierender Wirkung.

2. Verwendung der Verbindungen der Formel I gemäß Anspruch 1, worin R Phe-ol bedeutet, sowie deren physiologisch verträglichen Salze.

3. Verwendung der Verbindungen der Formel I oder II gemäß den Ansprüchen 1 und 2, wobei diese Verbindungen der Formel
AcPhe-Aib-Aib-Aib-Aib-Gly-Leu-Iva-Aib-Hyp-Gln-Iva-Hyp-Aib-Pro-Phe-ol;
AcPhe-Aib-Aib-Aib-Aib-Ala-Leu-Iva-Aib-Hyp-Gln-Iva-Hyp-Aib-Pro-Phe-ol;
AcPhe-Aib-Aib-Aib-Iva-Gly-Leu-Iva-Aib-Hyp-Gln-Iva-Hyp-Aib-Pro-Phe-ol;
AcPhe-Aib-Aib-Aib-Aib-Gly-Leu-Iva-Aib-Hyp-Gln-Aib-Hyp-Aib-Pro-Phe-ol;
AcPhe-Aib-Aib-Aib-Aib-Gly-Leu-Aib-Aib-Hyp-Gln-Aib-Hyp-Aib-Pro-Phe-ol;
AcPhe-Aib-Aib-Aib-Aib-Gly-Leu-Aib-Aib-Hyp-Gln-Iva-Hyp-Aib-Pro-Phe-ol;
AcPhe-Iva-Gln-Aib-Ile-Thr-Aib-Leu-Aib-Hyp-Gln-Aib-Hyp-Aib-Pro-Phe-Ser; oder
AcPhe-Iva-Gln-Aib-Ile-Thr-Aib-Leu-Aib-Pro-Gln-Aib-Hyp-Aib-Pro-Phe-Ser;
bedeuten sowie deren physiologisch verträglichen Salze.

4. Verwendung der Verbindungen der Formel I oder II gemäß den Ansprüchen 1 bis 3 zur Herstellung eines Medikaments zur Behandlung von chronischer Glomerulonephritis mit nephrotischen Syndrom, chronisch entzündlichen Darmkrankheiten, Myasthenia gravis, Autoimmunhepatitis, thrombopenische Purpura oder entzündlichen rheumatischen Erkrankungen oder zur Anwendung in der Transplantationsmedizin.


SEQUENZPROTOKOLL

<110> Sanofi-Aventis Deutschland GmbH

<120> Neue Verwendung von Cephaibolen

<130> DEAV 2002/0027

<140>
<141>

<150> 10222792.6
<151> 2002-05-23

<160> 8

<170> PatentIn Ver. 2.1

<210> 1
<211> 16
<212> PRT
<213> acremonium tubakii

<220>
<221> PEPTIDE
<222> (1)
<223> Xaa = AcPhe

<220>
<221> PEPTIDE
<222> (2)..(5)
<223> Xaa = Aib

<220>
<221> PEPTIDE
<222> (8)
<223> Xaa = Iva

<220>
<221> PEPTIDE
<222> (9)
<223> Xaa = Aib

<220>
<221> PEPTIDE
<222> (10)
<223> Xaa = Hyp

<220>
<221> PEPTIDE
<222> (12)
<223> Xaa = Iva

<220>
<221> PEPTIDE
<222> (13)
<223> Xaa= Hyp

<220>
<221> PEPTIDE
<222> (14)
<223> Xaa = Aib

<220>
<221> PEPTIDE
<222> (16)
<223> Xaa = Phe-ol

<400> 1
Xaa Xaa Xaa Xaa Xaa Gly Leu Xaa Xaa Xaa Gln Xaa Xaa Xaa Pro Xaa
1               5                   10                  15

<210> 2
<211> 16
<212> PRT
<213> acremonium tubakii

<220>
<221> PEPTIDE
<222> (1)
<223> Xaa = AcPhe

<220>
<221> PEPTIDE
<222> (2)..(4)
<223> Xaa = Aib

<220>
<221> PEPTIDE
<222> (5)
<223> Xaa = Iva

<220>
<221> PEPTIDE

<222> (8)
<223> Xaa = Iva

<220>
<221> PEPTIDE
<222> (9)
<223> Xaa = Aib

<220>
<221> PEPTIDE
<222> (10)
<223> Xaa = Hyp

<220>
<221> PEPTIDE
<222> (12)
<223> Xaa = Iva

<220>
<221> PEPTIDE
<222> (13)
<223> Xaa = Hyp

<220>
<221> PEPTIDE
<222> (14)
<223> Xaa = Aib

<220>
<221> PEPTIDE
<222> (16)
<223> Xaa = Phe-ol

<400> 2
Xaa Xaa Xaa Xaa Xaa Gly Leu Xaa Xaa Xaa Gln Xaa Xaa Xaa Pro Xaa
1               5                   10                  15

<210> 3
<211> 16
<212> PRT
<213> acremonium tubakii

<220>
<221> PEPTIDE
<222> (1)
<223> Xaa = AcPhe

<220>
<221> PEPTIDE
<222> (2)..(5)
<223> Xaa = Aib

<220>
<221> PEPTIDE
<222> (8)
<223> Xaa = Iva

<220>
<221> PEPTIDE
<222> (9)
<223> Xaa = Aib

<220>
<221> PEPTIDE
<222> (10)
<223> Xaa = Hyp

<220>
<221> PEPTIDE
<222> (12)
<223> Xaa = Aib

<220>
<221> PEPTIDE
<222> (13)
<223> Xaa = Hyp

<220>
<221> PEPTIDE
<222> (14)
<223> Xaa = Aib

<220>
<221> PEPTIDE
<222> (16)
<223> Xaa = Phe-ol

<400> 3
Xaa Xaa Xaa Xaa Xaa Gly Leu Xaa Xaa Xaa Gln Xaa Xaa Xaa Pro Xaa
1               5                   10                  15

<210> 4

<211> 16
<212> PRT
<213> acremonium tubakii

<220>
<221> PEPTIDE
<222> (1)
<223> Xaa = AcPhe

<220>
<221> PEPTIDE
<222> (2)..(5)
<223> Xaa = Aib

<220>
<221> PEPTIDE
<222> (8)..(9)
<223> Xaa = Aib

<220>
<221> PEPTIDE
<222> (10)
<223> Xaa = Hyp

<220>
<221> PEPTIDE
<222> (12)
<223> Xaa = Aib

<220>
<221> PEPTIDE
<222> (13)
<223> Xaa = Hyp

<220>
<221> PEPTIDE
<222> (14)
<223> Xaa = Aib

<220>
<221> PEPTIDE
<222> (16)
<223> Xaa = Phe-ol

<400> 4
Xaa Xaa Xaa Xaa Xaa Gly Leu Xaa Xaa Xaa Gln Xaa Xaa Xaa Pro Xaa
1               5                   10                  15

<210> 5
<211> 16
<212> PRT
<213> acremonium tubakii

<220>
<221> PEPTIDE
<222> (1)
<223> Xaa = AcPhe

<220>
<221> PEPTIDE
<222> (2)..(5)
<223> Xaa = Aib

<220>
<221> PEPTIDE
<222> (8)..(9)
<223> Xaa = Aib

<220>
<221> PEPTIDE
<222> (10)
<223> Xaa = Hyp

<220>
<221> PEPTIDE
<222> (12)
<223> Xaa = Iva

<220>
<221> PEPTIDE
<222> (13)
<223> Xaa = Hyp

<220>
<221> PEPTIDE
<222> (14)
<223> Xaa = Aib

<220>
<221> PEPTIDE
<222> (16)
<223> Xaa = Phe-ol

<400> 5

Xaa Xaa Xaa Xaa Xaa Gly Leu Xaa Xaa Xaa Gln Xaa Xaa Xaa Pro Xaa
1               5                   10                  15

<210> 6
<211> 17
<212> PRT
<213> acremonium tubakii

<220>
<221> PEPTIDE
<222> (1)
<223> Xaa = AcPhe

<220>
<221> PEPTIDE
<222> (2)
<223> Xaa = Iva

<220>
<221> PEPTIDE
<222> (4)
<223> Xaa = Aib

<220>
<221> PEPTIDE
<222> (7)
<223> Xaa = Aib

<220>
<221> PEPTIDE
<222> (9)
<223> Xaa = Aib

<220>
<221> PEPTIDE
<222> (10)
<223> Xaa = Hyp

<220>
<221> PEPTIDE
<222> (12)
<223> Xaa = Aib

<220>
<221> PEPTIDE
<222> (13)

<223> Xaa = Hyp

<220>
<221> PEPTIDE
<222> (14)
<223> Xaa = Aib

<400> 6
Xaa Xaa Gln Xaa Ile Thr Xaa Leu Xaa Xaa Gln Xaa Xaa Xaa Pro Phe
1               5                   10                  15

Ser

<210> 7
<211> 17
<212> PRT
<213> acremonium tubakii

<220>
<221> PEPTIDE
<222> (1)
<223> Xaa = AcPhe

<220>
<221> PEPTIDE
<222> (2)
<223> Xaa = Iva

<220>
<221> PEPTIDE
<222> (4)
<223> Xaa = Aib

<220>
<221> PEPTIDE
<222> (7)
<223> Xaa = Aib

<220>
<221> PEPTIDE
<222> (9)
<223> Xaa = Aib

<220>
<221> PEPTIDE

<222> (12)
<223> Xaa = Aib

<220>
<221> PEPTIDE
<222> (13)
<223> Xaa = Hyp

<220>
<221> PEPTIDE
<222> (14)
<223> Xaa = Aib

<400> 7
Xaa Xaa Gln Xaa Ile Thr Xaa Leu Xaa Pro Gln Xaa Xaa Xaa Pro Phe
1               5                   10                  15

Ser

<210> 8
<211> 16
<212> PRT
<213> acremonium tubakii

<220>
<221> PEPTIDE
<222> (1)
<223> Xaa = AcPhe

<220>
<221> PEPTIDE
<222> (2)..(5)
<223> Xaa = Aib

<220>
<221> PEPTIDE
<222> (8)
<223> Xaa = Iva

<220>
<221> PEPTIDE
<222> (9)
<223> Xaa = Aib

<220>

<221> PEPTIDE
<222> (10)
<223> Xaa = Hyp

<220>
<221> PEPTIDE
<222> (12)
<223> Xaa = Iva

<220>
<221> PEPTIDE
<222> (13)
<223> Xaa = Hyp

<220>
<221> PEPTIDE
<222> (14)
<223> Xaa = Aib

<220>
<221> PEPTIDE
<222> (16)
<223> Xaa = Phe-ol

<400> 8
Xaa Xaa Xaa Xaa Xaa Ala Leu Xaa Xaa Xaa Gln Xaa Xaa Xaa Pro Xaa
1               5                   10                  15